# FASCICULE DE BREVET EUROPEEN

(11) **EP 0 612 713 B1**
(45) Date de publication et mention de la délivrance du brevet: **18.06.1997**
(21) Numéro de dépôt: 94420043.5
(22) Date de dépôt: 10.02.1994
(51) Int. Cl.: C07C 57/03, C07C 51/12

(54) **Procédé de carbonylation des buténols allyliques et de leurs esters**
Verfahren zur Carbonylierung der Allylbutenole und ihrer Ester
Process for the carbonylation of allylic butenols and their esters

(30) Priorité: 26.02.1993 FR 9302482
(43) Date de publication de la demande: 31.08.1994
(73) Titulaire: RHONE-POULENC FIBER & RESIN INTERMEDIATES, 92405 Courbevoie (FR)
(72) Inventeur: Denis, Philippe, F-69150 Decines (FR); Metz, Francois, F-69540 Irigny (FR); Patois, Carl, F-69003 Lyon (FR); Perron, Robert, F-69390 Charly (FR)
(74) Mandataire: Vignally, Noel

(56) Documents cités:
- FR-A- 2 180 021
- US-A- 3 427 344

## Description

La présente invention concerne la carbonylation à l'aide de monoxyde de carbone des buténols allyliques et/ou de leurs esters carboxyliques.

Le brevet US-A-4 801 743 décrit la carbonylation de l'alcool allylique par le monoxyde de carbone, en présence d'un catalyseur au palladium et d'un promoteur choisi parmi le fluorure d'hydrogène, le chlorure d'hydrogène, le bromure d'hydrogène et l'iodure d'hydrogène.

Le brevet EP-A-0 428 979 décrit la carbonylation des buténols allyliques et de leurs esters, en présence d'un catalyseur au rhodium et d'un promoteur choisi parmi le bromure d'hydrogène et l'iodure d'hydrogène, le rapport molaire promoteur/rhodium étant compris entre 1/1 et 10/1.

Le brevet US-A-3 427 344 décrit la préparation d'esters d'acides carboxyliques insaturés par réaction d'un composé allylique, de préférence halogéné, avec un monoxyde de carbone et un éther cyclique, en présence d'un catalyseur choisi dans les groupes VII et VIII. Les catalyseurs utilisés sont essentiellement le palladium et le rhodium et le procédé est appliqué en pratique aux dérivés halogénés des composés allyliques.

Le brevet FR-A-2 180 021 décrit un procédé de préparation de l'acide vinylacétique et éventuellement de γ-butyrolactone, par réaction de l'alcool allylique, d'un éther allylique ou d'un ester allylique d'un acide carboxylique, avec le monoxyde de carbone, en présence de catalyseurs à base de métaux lourds des groupes VI, VII et VIII de la classification périodique des éléments.

Ces deux procédés de l'art antérieur ne permettent pas d'accéder directement aux acides penténoïques.

Il a maintenant été trouvé un nouveau procédé de carbonylation des buténols allyliques et/ou de leurs esters carboxyliques, consistant à faire réagir le monoxyde de carbone avec au moins un desdits buténols allyliques et esters carboxyliques, à température élevée, sous une pression supérieure à la pression atmosphérique et en présence d'un catalyseur à l'iridium et d'un promoteur iodé ou bromé, le rapport molaire promoteur/iridium étant compris entre 0,1/1 et 20/1.

L'activité des catalyseurs à l'iridium dans cette réaction est surprenante, car l'iridium n'est pas connu pour catalyser l'hydroxycarbonylation du butadiène, les catalyseurs au palladium ou au rhodium étant généralement utilisés pour cette réaction.

Les buténols allyliques sont le 3-butène-2-ol, le 2-butène-1-ol et leurs mélanges; les esters des buténols que l'on utilise dans le procédé de l'invention sont essentiellement les carboxylates de ces buténols et plus particulièrement ceux qui dérivent des acides carboxyliques aliphatiques, saturés ou insaturés, ayant 1 à 12 atomes de carbone. A titre d'exemples non limitatifs, on peut citer les esters de buténol allylique de l'acide acétique, de l'acide propionique, de l'acide valérique, de l'acide adipique, des acides penténoïques (acides 2-penténoïque, 3-penténoïque et 4-penténoïque).

Préférentiellement on mettra en oeuvre comme substrat les buténols allyliques et/ou leurs acétates, leurs valérates ou leurs penténoates. En pratique il est préférable, lorsque l'on utilise un ester de buténol, de choisir les esters des acides carboxyliques susceptibles de se former au cours de la réaction de carbonylation, comme l'acide valérique ou l'acide 3-penténoïque.

Les buténols allyliques sont des composés disponibles dans le commerce; ils peuvent également être facilement préparés par hydratation du butadiène. Leurs esters peuvent être obtenus par réaction desdits buténols ou du butadiène avec l'acide carboxylique choisi. Ces préparations des substrats peuvent être réalisées extemporanément ou dans le réacteur de carbonylation lui-même.

Pour le catalyseur à l'iridium nécessaire dans le présent procédé, diverses sources d'iridium sont susceptibles d'être mises en oeuvre.

A titre d'exemples de telles sources d'iridium, on peut citer :
- Ir métallique; IrO₂ ; Ir₂O₃ ;
- IrCl₃ ; IrCl₃, 3H₂O ;
- IrBr₃ ; IrBr₃, 3H₂O ;
- IrI₃ ;
- Ir₂(CO)₄Cl₂ ; Ir₂(CO)₄I₂ ;
- Ir₂(CO)₈ ; Ir₄(CO)₁₂ ;
- Ir(CO)[P(C₆H₅)₃]₂I ;
- Ir(CO)[P(C₆H₅)₃]₂Cl ;
- Ir[P(C₆H₅)₃]₃I ;
- HIr[P(C₆H₅)₃]₃(CO) ;
- Ir(acac)(CO)₂ ;
- [IrCl(cod)]₂ ;
(acac = acétylacétonate ; cod = cyclooctadiène-,5).

Les catalyseurs à l'iridium qui conviennent plus particulièrement sont: [IrCl(cod)]₂, Ir₄(CO)₁₂ et Ir(acac)(CO)₂.

La quantité de catalyseur à mettre en oeuvre peut varier dans de larges limites.

En général, une quantité, exprimée en moles d'iridium métal par litre de mélange réactionnel, comprise entre 10⁻⁴et 10⁻¹ conduit à des résultats satisfaisants. Des quantités inférieures peuvent être utilisées, mais on observe toutefois que la vitesse de réaction est faible. Des quantités supérieures n'ont des inconvénients qu'au plan économique.

De préférence, la concentration en iridium dans le mélange réactionnel est comprise entre 5.10⁻⁴et 5.10⁻² mole/litre.

Par promoteur iodé ou bromé, on entend dans le cadre du procédé l'iodure d'hydrogène, le bromure d'hydrogène et les composés organo-iodés et organo-bromés capables de générer respectivement l'iodure d'hydrogène et le bromure d'hydrogène dans les conditions réactionnelles; ces composés organo-iodés et organo-bromés sont plus particulièrement les iodures et les bromures d'alkyle ayant de 1 à 10 atomes de carbone, parmi lesquels l'iodure de méthyle et le bromure de méthyle sont préférés.

Le rapport molaire promoteur/lr est de préférence compris entre 1/1 et 10/1.

On peut également compléter le système catalytique constitué par le catalyseur proprement dit à l'iridium et le promoteur par une quantité efficace d'un copromoteur tel qu'un iodure métallique ou un bromure métallique ou encore un carboxylate métallique susceptible de se transformer au moins partiellement en iodure ou bromure correspondant dans le milieu réactionnel. Ce copromoteur peut être notamment un iodure ou un bromure de métal alcalin ou un acétate de métal alcalin. Ainsi il est favorable, en particulier au plan de l'activité du catalyseur, d'utiliser l'iodure de lithium, l'iodure de sodium, le bromure de lithium, le bromure de sodium, I'acétate de lithium ou l'acétate de sodium. Le rapport molaire copromoteur/promoteur peut varier très largement, par exemple de 0,01/1 à 100/1 et de préférence de 0,1/1 à 50/1.

La réaction est conduite en phase liquide. La température à laquelle on opère est généralement de 50°C à 250°C et de préférence de 80°C à 200°C.

La pression totale à la température de la réaction peut varier dans de larges limites. La pression partielle du monoxyde de carbone, mesurée à 25°C, est généralement de 0,5 bar à 100 bars et de préférence de 1 bar à 50 bars. Le monoxyde de carbone utilisé peut être le monoxyde de carbone sensiblement pur ou le monoxyde de carbone de qualité technique, tels qu'ils se trouvent dans le commerce.

La réaction de carbonylation des buténols allyliques et/ou de leurs esters est généralement conduite dans un solvant.

Comme solvant, on peut utiliser notamment les acides carboxyliques aliphatiques, saturés ou insaturés, ou aromatiques, comportant au plus 20 atomes de carbone, dans la mesure où ils sont liquides dans les conditions réactionnelles. A titre d'exemples de tels acides carboxyliques, on peut citer l'acide acétique, l'acide propionique, l'acide butanoïque, l'acide valérique, l'acide adipique, les acides penténoïques, l'acide benzoïque et l'acide phénylacétique. Lorsque l'on carbonyle un ester de buténol allylique, il est judicieux d'utiliser comme solvant l'acide carboxylique correspondant à l'ester.

D'autres familles de solvants peuvent également être utilisés, notamment les hydrocarbures saturés aliphatiques ou cycloaliphatiques et leurs dérivés chlorés, dans la mesure où ils sont liquides dans les conditions réactionnelles. A titre d'exemples de tels solvants, on peut citer le benzène, le toluène, le chlorobenzène, le dichlorométhane, l'hexane et le cyclohexane.

Lorsqu'il est présent dans le mélange réactionnel, le solvant représente par exemple de 10 % à 99 % en volume par rapport au volume total dudit mélange réactionnel et de préférence de 30 % à 90 % en volume.

Le procédé de carbonylation selon l'invention permet de préparer les acides penténoïques, en particulier l'acide 3-penténoïque sous ses formes isomériques cis et trans et dans une moindre mesure l'acide 2-penténoïque. Ces acides penténoïques peuvent être eux-mêmes hydroxycarbonylés par le monoxyde de carbone et l'eau en acide adipique, l'une des matières premières de la préparation des polyamides 6-6.

L'hydroxycarbonylation des acides penténoïques pouvant être effectuée avantageusement en présence d'un catalyseur à l'iridium et d'un promoteur iodé ou bromé, on peut, le cas échéant, procéder à cette deuxième réaction directement sur le mélange réactionnel issu de la carbonylation des buténols allyliques et/ou de leurs esters ou après une séparation sommaire des sous-produits de la réaction, en utilisant le même appareillage. Il est également possible de traiter le mélange réactionnel selon toute technique appropriée pour séparer les acides penténoïques obtenus, le système catalytique, I'éventuel solvant et les divers sous-produits formés.

Le procédé de l'invention peut être mis en oeuvre en continu ou en discontinu.

Les exemples qui suivent illustrent l'invention.

### EXEMPLE 1

Dans un autoclave de 125 cm³ ,préalablement purgé à l'argon, on introduit successivement:

| | |
|---|---|
| - valérate de crotyle (pentanoate de 2-butényle) : | 50 mmol |
| - acide valérique (PA) : | 46 cm³ |
| - iodure d'hydrogène (en solution aqueuse à 57 % en poids) : | 1,5 mmol |
| - [IrCl(cod)]₂ : | 0,5 mmol |
| - Lil : | 15 mmol |

L'autoclave est fermé, placé dans un four agité et raccordé à l'alimentation de CO sous pression. On met 5 bar de CO à 25 °C, puis on chauffe à 140 °C. On ajuste la pression en température à 50 bar à l'aide de CO (ce qui correspond à 36 bar de pression partielle de CO mesurée à 25 °C) et cette pression est maintenue pendant 2 heures à 140 °C.

On refroidit ensuite l'autoclave, on le dégaze et le mélange réactionnel est analysé par chromatographie en phase gazeuse (CPG).

On obtient les résultats suivants:

| | |
|---|---|
| - taux de transformation (TT) du valérate de crotyle : | 35 % |
| - rendement en acide 3-penténoïque (P3) par rapport au valérate de crotyle chargé (RR) : | 24 % |

D'autres composés ont été identifiés par CPG :
- l'acide 2-méthyl-3-buténoïque (MBE)
- l'acide 2-méthyl-butanoïque (MBA)
- des butènes (C4)
- l'acide valérique.

### EXEMPLE 2

On reproduit l'exemple 1 en remplaçant l'iodure d'hydrogène et l'iodure de lithium par 1,5 mmol de bromure d'hydrogène.

On obtient les résultats suivants :

| | |
|---|---|
| - TT du valérate de crotyle : | 80 % |
| - RR en P3 : | 40 % |

### EXEMPLES 3 à 7

On reproduit l'exemple 1 en utilisant des quantités variables d'iodure d'hydrogène et d'iodure de lithium (indiquées dans le tableau ci-après). En outre l'exemple 7 a été réalisé à 185°C au lieu de 140°C. Les résultats obtenus sont rassemblés dans le tableau ci-après.

| Exemples | Hl (mmol) | LiI (mmol) | TT % du valérate de crotyle | RR % P3 | RR % MBE + MBA | RR % C4 |
|---|---|---|---|---|---|---|
| Exemple 3 | 1,5 | 0 | 48 | 16 | 20 | 15 |
| Exemple 4 | 4,0 | 15 | 63 | 29 | 5 | 7 |
| Exemple 5 | 0,5 | 15 | 48 | 10 | 4 | 6 |
| Exemple 6 | 1,5 | 30 | 70 | 24 | 4 | 12 |
| Exemple 7 | 1,5 | 0 | 80 | 17 | 13 | 18 |

### EXEMPLE 8

On reproduit l'exemple 1 en remplaçant le valérate de crotyle par le 2-butène-1-ol (ou alcool crotylique) et en supprimant l'iodure de lithium.

On obtient les résultats suivants :

| | |
|---|---|
| - TT du 2-butène-1-ol : | 100 % |
| - RR en P3 : | 24 % |

### EXEMPLE 9

Dans un autoclave de 125 cm³ ,préalablement purgé à l'argon, on introduit successivement:

| | |
|---|---|
| - 3-penténoate de 2-butényle : | 50 mmol |
| - acide acétique : | 46 cm³ |
| - bromure d'hydrogène : | 1,5 mmol |
| - [IrCl(cod)]₂ : | 0,5 mmol |
| - NaCH₃COO : | 3 mmol |

L'autoclave est fermé, placé dans un four agité et raccordé à l'alimentation de CO sous pression. On met 5 bar de CO à 25°C, puis on chauffe à 140°C. On ajuste la pression en température à 50 bar à l'aide de CO (ce qui correspond à 36 bar de pression partielle de CO mesurée à 25 °C) et cette pression est maintenue pendant 1 heure 30 à 140°C.

On refroidit ensuite l'autoclave, on le dégaze et le mélange réactionnel est analysé par chromatographie en phase gazeuse (CPG).

On obtient les résultats suivants:

| | |
|---|---|
| - TT du 3-penténoate de 2-butényle : | 31 % |
| - RR en P3 : | 15 % |
| - RR en C4 : | 4 % |
| - RR en butadiène : | 1 % |
| - RR en diacides en C6 : | 1 % |

### ESSAI COMPARATIF 1

Dans un autoclave de 125 cm³ ,préalablement purgé à l'argon, on introduit successivement:

| | |
|---|---|
| - butadiène : | 80 mmol |
| - eau : | 90 mmol |
| - acide valérique : | 46 cm³ |
| - iodure d'hydrogène : | 1,5 mmol |
| - [IrCl(cod)]₂ : | 0,5 mmol |

L'autoclave est fermé, placé dans un four agité et raccordé à l'alimentation de CO sous pression. On met 5 bar de CO à 25 °C, puis on chauffe à 140 °C. On ajuste la pression en température à 50 bar à l'aide de CO (ce qui correspond à 36 bar de pression partielle de CO mesurée à 25 °C) et cette pression est maintenue pendant 2 heures à 140 °C.

On refroidit ensuite l'autoclave, on le dégaze et le mélange réactionnel est analysé par chromatographie en phase gazeuse (CPG).

On obtient les résultats suivants:

| | |
|---|---|
| - TT du butadiène : | 100 % |
| - RR en P3 : | 6 % |
| - RR en C4 : | 32 % |
| - RR en acide valérique : | 6 % |
| - RR en MBA + MBE : | 9 % |

## Revendications

1. Procédé de carbonylation en acides penténoïques des buténols allyliques et/ou de leurs esters carboxyliques, caractérisé en ce que l'on fait réagir le monoxyde de carbone avec au moins un desdits buténols allyliques et esters carboxyliques, à température élevée, sous une pression supérieure à la pression atmosphérique et en présence d'un catalyseur à l'iridium et d'un promoteur iodé ou bromé, le rapport molaire promoteur/iridium étant compris entre 0,1/1 et 20/1.

2. Procédé selon la revendication 1, caractérisé en ce que les buténols allyliques sont le 3-butène-2-ol, le 2-butène-1-ol et leurs mélanges et les esters des buténols sont les carboxylates de ces buténols qui dérivent des acides carboxyliques aliphatiques, saturés ou insaturés, ayant 1 à 12 atomes de carbone.

3. Procédé selon l'une des revendications 1 ou 2, caractérisé en ce que les esters de buténol allylique mis en oeuvre sont les esters de buténol allylique de l'acide acétique, de l'acide propionique, de l'acide valérique, de l'acide adipique, des acides penténoïques.

4. Procédé selon l'une des revendications 1 à 3, caractérisé en ce que le catalyseur à l'iridium est choisi parmi les diverses sources d'iridium suivantes :
- Ir métallique; IrO₂; Ir₂O₃;
- IrCl₃; IrCl₃, 3H₂O;
- IrBr₃; IrBr₃, 3H₂O;
- IrI₃;
- Ir₂(CO)₄Cl₂; Ir₂(CO)₄I₂;
- Ir₂(CO)₈; Ir₄(CO)₁₂;
- Ir(CO)[P(C₆H₅)₃]₂I;
- Ir(CO)[P(C₆H₅)₃]₂Cl;
- Ir[P(C₆H₅)₃]₃I;
- HIr[P(C₆H₅)₃]₃(CO);
- Ir(acac)(CO)₂;
- [IrCl(cod)]₂.

5. Procédé selon l'une des revendications 1 à 4, caractérisé en ce que la quantité de catalyseur à l'iridium mise en oeuvre, exprimée en moles d'iridium métal par litre de mélange réactionnel, est comprise entre 10⁻⁴ et 10⁻¹ et est de préférence comprise entre 5.10⁻⁴ et 5.10⁻² mole/litre.

6. Procédé selon l'une des revendications 1 à 5, caractérisé en ce que le promoteur iodé ou bromé est choisi parmi l'iodure d'hydrogène, le bromure d'hydrogène et les composés organo-iodés et organo-bromés capables de générer respectivement l'iodure d'hydrogène et le bromure d'hydrogène dans les conditions réactionnelles.

7. Procédé selon l'une des revendications 1 à 6, caractérisé en ce que le rapport molaire promoteur/lr est de préférence compris entre 1/1 et 10/1.

8. Procédé selon l'une des revendications 1 à 7, caractérisé en ce que l'on complète le système catalytique constitué par le catalyseur proprement dit à l'iridium et le promoteur par une quantité efficace d'un copromoteur tel qu'un iodure métallique ou un bromure métallique, ou un carboxylate métallique susceptible de se transformer au moins partiellement en iodure ou bromure correspondant dans le milieu réactionnel, de préférence un iodure ou un bromure de métal alcalin ou un acétate de métal alcalin.

9. Procédé selon la revendication 8, caractérisé en ce que le rapport molaire copromoteur/promoteur est de 0,01/1 à 100/1 et de préférence de 0,1/1 à 50/1.

10. Procédé selon l'une des revendications 1 à 9, caractérisé en ce que la réaction est conduite en phase liquide, à une température de 50°C à 250°C et de préférence de 80°C à 200°C et sous une pression partielle du monoxyde de carbone, mesurée à 25°C, de 0,5 bar à 100 bars et de préférence de 1 bar à 50 bars.

11. Procédé selon l'une des revendications 1 à 10, caractérisé en ce que la réaction de carbonylation est conduite dans un solvant choisi parmi les acides carboxyliques aliphatiques, saturés ou insaturés, ou aromatiques, comportant au plus 20 atomes de carbone, les hydrocarbures saturés aliphatiques ou cycloaliphatiques et leurs dérivés chlorés, liquides dans les conditions réactionnelles.

12. Procédé selon la revendication 11, caractérisé en ce que le solvant est choisi parmi l'acide acétique, l'acide propionique, l'acide butanoïque, l'acide valérique, l'acide adipique, les acides penténoïques, l'acide benzoïque, l'acide phénylacétique, le benzène, le toluène, le chlorobenzène, le dichlorométhane, l'hexane et le cyclohexane.

13. Procédé selon l'une des revendications 11 ou 12, caractérisé en ce que le solvant représente de 10 % à 99 % en volume par rapport au volume total du mélange réactionnel et de préférence de 30 % à 90 % en volume.

## Claims

1. Process for the carbonylation into pentenoic acids of allylic butenols and/or their carboxylic esters, characterized in that carbon monoxide is reacted with at least one of the said allylic butenols and carboxylic esters, at elevated temperature, under a pressure greater than atmospheric pressure, and in the presence of an iridium catalyst and of an iodinated or brominated promoter, the promoter/iridium molar ratio being between 0.1:1 and 20:1.

2. Process according to Claim 1, characterized in that the allylic butenols are 3-buten-2-ol, 2-buten-1-ol and their mixtures, and the butenol esters are the carboxylates of these butenols which are derived from saturated or unsaturated aliphatic carboxylic acids having 1 to 12 carbon atoms.

3. Process according to one of Claims 1 and 2, characterized in that the allylic butenol esters used are the allylic butenol esters of acetic acid, of propionic acid, of valeric acid, of adipic acid or of pentenoic acids.

4. Process according to one of Claims 1 to 3, characterized in that the iridium catalyst is chosen from the following various sources of iridium:
- metallic Ir; IrO₂; Ir₂O₃;
- IrCl₃; IrCl₃.3H₂O;
- IrBr₃; IrBr₃.3H₂O;
- IrI₃;
- Ir₂(CO)₄Cl₂; Ir₂(CO)₄I₂;
- Ir₂(CO)₈; Ir₄(CO)₁₂;
- Ir(CO)[P(C₆H₅)₃]₂I;
- Ir(CO)[P(C₆H₅)₃]₂Cl;
- Ir[P(C₆H₅)₃]₃I;
- HIr[P(C₆H₅)₃]₃(CO);
- Ir(acac)(CO)₂;
- [IrCl(cod)]₂.

5. Process according to one of Claims 1 to 4, characterized in that the quantity of iridium catalyst used, expressed in moles of iridium metal per litre of reaction mixture, is between 10⁻⁴ and 10⁻¹, and is preferably between 5x10⁻⁴ and 5x10⁻² mol/litre.

6. Process according to one of Claims 1 to 5, characterized in that the iodinated or brominated promoter is chosen from hydrogen iodide, hydrogen bromide and the organoiodine and organobromine compounds which are capable of generating respectively hydrogen iodide and hydrogen bromide under the reaction conditions.

7. Process according to one of Claims 1 to 6, characterized in that the promoter/Ir molar ratio is preferably between 1:1 and 10:1.

8. Process according to one of Claims 1 to 7, characterized in that the catalyst system consisting of the actual iridium catalyst and the promoter is complemented with an effective quantity of a copromoter such as a metal iodide or a metal bromide, or a metal carboxylate which is capable of being converted, at least partially, into the corresponding iodide or bromide in the reaction mixture, preferably an alkali metal iodide or bromide or an alkali metal acetate.

9. Process according to Claim 8, characterized in that the copromoter/promoter molar ratio is from 0.01:1 to 100:1 and preferably from 0.1:1 to 50:1.

10. Process according to one of Claims 1 to 9, characterized in that the reaction is carried out in liquid phase, at a temperature of from 50°C to 250°C and preferably from 80°C to 200°C and at a partial pressure of carbon monoxide, measured at 25°C, of from 0.5 bar to 100 bars, and preferably from 1 bar to 50 bars.

11. Process according to one of Claims 1 to 10, characterized in that the carbonylation reaction is carried out in a solvent chosen from saturated or unsaturated aliphatic or aromatic carboxylic acids containing not more than 20 carbon atoms, saturated aliphatic or cycloaliphatic hydrocarbons and their chlorinated derivatives which are liquid under the reaction conditions.

12. Process according to Claim 11, characterized in that the solvent is chosen from acetic acid, propionic acid, butanoic acid, valeric acid, adipic acid, pentenoic acids, benzoic acid, phenylacetic acid, benzene, toluene, chlorobenzene, dichloromethane, hexane and cyclohexane.

13. Process according to one of Claims 11 and 12, characterized in that the solvent represents from 10 % to 99 % by volume relative to the total volume of the reaction mixture, and preferably from 30 % to 90 % by volume.

## Patentansprüche

1. Verfahren zur Carbonylierung von Allylbutenolen und/oder ihren Carbonsäureestern zu Pentensäuren, dadurch gekennzeichnet, daß man Kohlenmonoxid mit mindestens einem der genannten Allylbutenole und Carbonsäureester bei erhöhter Temperatur, unter einem höheren Druck als dem atmosphärischen Druck und in Anwesenheit eines Iridium-Katalysators und eines iodierten oder bromierten Promotors zur Reaktion bringt, wobei das molare Verhältnis Promotor/Iridium zwischen 0,1/1 und 20/1 beträgt.

2. Verfahren nach Anspruch 1, dadurch gekennzeichnet, daß die Allylbutenole das 3-Buten-2-ol, das 2-Buten-1-ol und ihre Mischungen sind und die Ester der Butenole die Carbonsäureester dieser Butenole darstellen, die sich von aliphatischen, gesättigten oder ungesättigten Carbonsäuren mit 1 bis 12 Kohlenstoffatomen ableiten.

3. Verfahren nach einem der Ansprüche 1 oder 2, dadurch gekennzeichnet, daß die eingesetzten Allylbutenol-Ester die Ester von Allylbutenol mit Essigsäure, Propionsäure, Valeriansäure, Adipinsäure und den Pentensäuren sind.

4. Verfahren nach einem der Ansprüche 1 bis 3, dadurch gekennzeichnet, daß der Iridium-Katalysator unter den verschiedenen, nachfolgend aufgeführten Iridiumquellen ausgewählt wird:
- metallisches Ir, IrO₂; Ir₂O₃;
- IrCl₃; IrCl₃, 3H₂O;
- IrBr₃; IrBr₃,3H₂O;
- IrI₃;
- Ir₂(CO)₄Cl₂; Ir₂(CO)₄I₂;
- Ir₂(CO)₈; Ir₄(CO)₁₂;
- Ir(CO)[P(C₆H₅)₃]₂I;
- Ir(CO)[P(C₆H₅)₃]₂Cl;
- Ir[P(C₆H₅)₃]₃I;
- HIr[P(C₆H₅)₃]₃(CO);
- Ir(acac)(CO)₂;
- [IrCl(cod)]₂.

5. Verfahren nach einem der Ansprüche 1 bis 4, dadurch gekennzeichnet, daß die Menge des eingesetzten Iridium-Katalysators, ausgedrückt in Molen Iridium-Metall pro Liter Reaktionsmischung, zwischen 10⁻⁴ und 10⁻¹ und vorzugsweise zwischen 5 · 10⁻⁴ und 5 · 10⁻² Mol/l beträgt.

6. Verfahren nach einem der Ansprüche 1 bis 5, dadurch gekennzeichnet, daß der iodierte oder bromierte Promotor ausgewählt wird unter Iodwasserstoff, Bromwasserstoff und den Organo-Iod- und Organo-Brom-Verbindungen, die fähig sind, unter den Reaktionsbedingungen Iodwasserstoff bzw. Bromwasserstoff zu erzeugen.

7. Verfahren nach einem der Ansprüche 1 bis 6, dadurch gekennzeichnet, daß das molare Verhältnis Promotor/Ir vorzugsweise zwischen 1/1 und 10/1 beträgt.

8. Verfahren nach einem der Ansprüche 1 bis 7, dadurch gekennzeichnet, daß man das aus dem eigentlichen Iridium-Katalysator und dem Promotor bestehende katalytische System durch eine wirksame Menge eines Co-Promotors vervollständigt, wie eines metallischen Iodids oder eines metallischen Bromids oder eines metallischen Carboxylats, das fähig ist, sich mindestens teilweise in dem Reaktionsmedium in das entsprechende Iodid oder Bromid umzuwandeln, vorzugsweise in ein Iodid oder ein Bromid eines Alkalimetalls oder in ein Alkalimetall-Acetat.

9. Verfahren nach Anspruch 8, dadurch gekennzeichnet, daß das molare Verhältnis Co-Promotor/Promotor 0,01/1 bis 100/1, und vorzugsweise 0,1/1 bis 50/1 beträgt.

10. Verfahren nach einem der Ansprüche 1 bis 9, dadurch gekennzeichnet, daß die Reaktion in flüssiger Phase bei einer Temperatur von 50 °C bis 250 °C, vorzugsweise von 80 °C bis 200 °C, und unter einem Partialdruck des Kohlenmonoxids, gemessen bei 25 °C, von 0,5 bar bis 100 bar, vorzugsweise von 1 bar bis 50 bar, durchgeführt wird.

11. Verfahren nach einem der Ansprüche 1 bis 10, dadurch gekennzeichnet, daß die Reaktion der Carbonylierung in einem Lösungsmittel durchgeführt wird, ausgewählt unter den gesättigten oder ungesättigten aliphatischen oder den aromatischen Carbonsäuren mit höchstens 20 Kohlenstoffatomen, den gesättigten aliphatischen oder cycloaliphatischen Kohlenwasserstoffen und ihren chlorierten Derivaten, die unter den Reaktionsbedingungen flüssig sind.

12. Verfahren nach Anspruch 11, dadurch gekennzeichnet, daß das Lösungsmittel unter Essigsäure, Propionsäure, Butansäure, Valeriansäure, Adipinsäure, den Pentensäuren, Benzoesäure, Phenylessigsäure, Benzol, Toluol, Chlorbenzol, Dichlormethan, Hexan und Cyclohexan ausgewählt wird.

13. Verfahren nach einem der Ansprüche 11 oder 12, dadurch gekennzeichnet, daß das Lösungsmittel 10 Vol.-% bis 99 Vol.-%, vorzugsweise 30 Vol.-% bis 90 Vol.-% darstellt, bezogen auf das Gesamtvolumen der Reaktionsmischung.
